# EUROPEAN PATENT SPECIFICATION

(11) **EP 1 348 699 B1**
(45) Date of publication and mention of the grant of the patent: **05.05.2010**
(21) Application number: 02252146.2
(22) Date of filing: 25.03.2002
(51) Int. Cl.: C07D 213/61

(54) **Process for the preparation of 2-chloro-5-methylpyridine-3-carbaldehyde**
Verfahren zur Herstellung von 2-Chloro-5-methylpyridin-3-carbaldehyd
Procédé pour la préparation de 2-chloro-5-méthyl-pyridine-3-carbaldehyde

(43) Date of publication of application: 01.10.2003
(73) Proprietor: Council of Scientific and Industrial Research, New Delhi 110 001 (IN)
(72) Inventor: Chinaraju, Bhimapaka, Indian Institute of Chemical Technology, Andhra Pradesh (IN); Rao, Vaidya Jayathirtha, Indian Institute of Chemical Technology, Andhra Pradesh (IN); Raghavan, Kondapuram Vijaya, Indian Institute of Chemical Technology, Andhra Pradesh (IN)
(74) Representative: Towler, Philip Dean

(56) References cited:
- EP-A- 0 748 798
- METH-COHN, OTTO ET AL: "A versatile new synthesis of quinolines and related fused pyridines. Par 12. A general synthesis of 2-chloropyridines and 2-pyridones" J. CHEM. SOC., PERKIN TRANS. I, no. 6, 1984, pages 1173-1182, XP000602179

## Description

The present invention relates to an improved process for the preparation of 2-chloro-5-methyl pyridine-3-carbaldehyde, a useful intermediate for the variety of pharmaceutical and pesticide products. This study pertains to the preparation of 2-chloro-5-methyl pyridine-3-carbaldehyde having formula **1** as shown below.

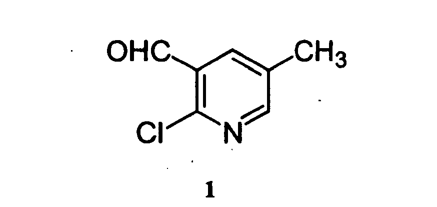

This study pertains to the preparation of 2-chloro-5-methyl pyridine-3-carbaldehyde in high yield.

### BACKGROUND OF THE INVENTION

Several pyridine, quinoline, and isoquinoline compounds are important intermediates in that they have been reported as potential anti tumor agents (J.Med.Chem, 19,1209,1976; CA,85:116544q). Carbaldehyde derived products served as ulcer healing agents (DE-OS-3324034,1984; CA,101:54936g), allergy inhibitors (EP 120483,1984; CA,102:95649e; EP120484,1984; CA,102:113500f), antiviral agents (CA,113:172015b), and useful as intermediates for pharmaceuticals (DE-0S-4429465,1996,CA, 124:343116u).

J.C.S. Perkin. Trans 1, 1173, 1984 describes a method for the preparation of 2-chloro-5-methylpyridine-3-carbaldehyde. According to this method 2-chloro-5-methylpyridine-3-carbaldehyde is produced in only 12% yield.

EP-A-1346984, which is part of the state of the art pursuant to Article 54(3) and(4) EPC, discloses a process for the preparation of 2-chloro-5-methylpyridine-3-carbaldehyde by reacting N-benzyl-N-(1-Propenyl) acetamide with a Vilsmeier reagent made by mixing dimethylformamide with diphosgene or triphosgene under dry conditions at a temperature in the range of 75-100°C.

The present invention describes an improved process for the preparation of 2-chloro-5-methylpyridine-3-carbaldehyde from N-benzyl-N-(1-Propenyl) acetamide using Vilsmeier reagent. The process according to the present invention results in a 2chloro-5-methylpyridine-3-carbaldehyde yield of 64%.

### OBJECT OF THE INVENTION

The main objective of the present invention is to provide an improved process for the preparation of 2-chloro-5-methylpyridine-3-carbaldehyde

Another objective of this present invention is to prepare 2-chloro-5-methylpyridine-3-carbaldehyde in high yield.

### SUMMARY OF THE INVENTION

Accordingly the present invention provides an improved process for the preparation of 2-chloro-5-methylpyridine-3-carbaldehyde which comprises reacting N-benzyl-N-(1-propenyl) acetamide with Vilsmeier reagent in a molar ratio of N-benzyl-N-(1-propenyl) acetamide to Vilsmeier reagent in the range of 1:17.5 to 1:5, at a temperature in the range of 0-25°C, preferably 0-10 °C, for 1-4 hrs, and heating the resulting reaction mixture at a temperature ranging between 70°C - 100°C for 4-6 hrs. Extraction may then be effected with a suitable solvent e.g. methylene chloride, whereby separation of the organic layer and removal of solvent enables one to obtain the desired product.

In an embodiment of the present invention the molar ratio of N-benzyl-N-(1-propenyl) acetamide to Vilsmeier reagent is preferably about 1:7.

In another embodiment the N-benzyl-N-(1-propenyl) acetamide and Vilsmeier reagent are reacted at 0 to 25°C for 2 hours. In yet another embodiment the reaction is heated to 75-90°C.

The process of the invention allows the desired product to be obtained in a yield of up to 64%.

### DETAILED DESCRIPTION

In the improved process for the preparation of the 2-chloro-5-methylpyridine-3-carbaldehyde, Vilsmeier reagent (chloromethylene dimethylammonium chloride) is prepared in situ by reacting phosphorus oxytrichloride with dimethylformamide at a temperature range of about 0-20°C for about 25-35 minutes. N-benzyl-N-(1-propenyl) acetamide is added to the same reaction pot. No additional solvent is required. The reaction conditions are carefully controlled: in a first stage, the reaction mixture is maintained at relatively low to ambient temperature and in a second stage, the reaction mixture is heated and maintained at elevated temperature. The resulting orange yellow organic mass is poured over ice cold water and extracted with organic solvent The organic layer is dried and the solvent removed.

The residue obtained is subjected to chromatographic purification over silicagel to give 2-chloro-5-methylpyridine-3-carbaldehyde.

The following examples are given by way of illustration of the present invention and therefore should not be construed to limit the scope of the present invention.

**Example 1** : Dimethylformamide (21.93 g, 0.3 moles) was added to well stirred and cooled phosphorus oxytrichloride (53.65 g, 0.35 moles) at 0°C in 30 minutes and followed by N-benzyl-N-(1-propenyl) acetamide (9.45g, 0.05 moles) at the same temperature . The reaction mixture was maintained for 2 hours at room temperature of about 25°C. The cooling mechanism was removed and the reaction mixture was heated to 75°C for 5 hours. The orange-yellow coloured organic mass was poured on to ice cold water (200g) with stirring .The mass was extracted with methylenechloride (2x 200ml) and the layers were separated. The organic layer was dried over sodium sulfate, and the solvent was removed under reduced pressure . The obtained residue was subjected to chromatographic purification on silicagel to give 2-chloro-5-methylpyridine-3-carbaldehyde in 64% yield .

**Example 2** : Dimethylformamide (21.93 g, 0.3moles) was added to well stirred and cooled phosphorus oxytrichloride (53.65 g, 0.35 moles) at 10 °C in 30 minutes and followed by N-benzyl-N-(1-propenyl) acetamide (9.45g, 0.05 moles) at the same temperature . The reaction mixture was maintained for 2 hours at room temperature of about 25°C. The cooling mechanism was removed and the reaction mixture was heated to 90°C for 5 hours. The work up procedure was carried out according to the above mentioned procedure. The obtained residue was subjected to chromatographic purification on silica gel to give 2-chloro-5-methylpyridine-3-carbaldehyde in 64% yield.

### Advantages : The various advantages of the method are given below.

1. The main advantage of the present invention is that 2-chloro-5-methylpyridine-3-carbaldehyde is produced in much higher yields than have previously been achieved.
2. The advantage of the present invention is the employment of controlled time and temperature conditions during the reaction which are critical to the formation of the reagent and the product.
3. Another advantage of the present invention is that it does not require any other solvent, long reaction times or harsh reaction temperatures.
4. Product isolation is straightforward.

## Claims (Claims for the following Contracting State(s): DE, FR, GB)

1. A process for the preparation of 2-chloro-5-methylpyridine-3-carbaldehyde which comprises reacting N-benzyl-N-(1-propenyl) acetamide with Vilsmeier reagent in a molar ratio of N-benzyl-N-(1-propenyl) acetamide to Vilsmeier reagent in the range of 1:17.5 to 1:5 at a first temperature in the range of 0-25°C for 1-4 hrs, and heating the resulting reaction mixture at a second temperature in the range of 70-100°C for 4-6 hrs, but not including a process comprising reacting N-benzyl-N-( 1-propenyl) acetamide with a reagent selected from dimethylformamide mixed with diphosgene or triphosgene under dry conditions at a temperature in the range of 75-100°C.

2. A process as claimed in claim 1 comprising extracting the reaction product of the heating stage with methylene chloride, separating the organic layer and removing the solvent to obtain the desired product.

3. A process as claimed in claim 1 or claim 2, wherein the mole ratio of N-benzyl-N-(1-propenyl) acetamide to Vilsmeier reagent is about 1:7.

4. A process as claimed in any one of claims 1 to 3, wherein N-benzyl-N-(1-propenyl) acetamide and Vilsmeier reagent are reacted at the first temperature for 2 hours.

5. A process as claimed in any one of claims 1 to 4, wherein the second temperature is in the range of 75-90 °C.

6. A process as claimed in any one of claims 1 to 5, wherein the first temperature is in the range of 0-10 °C.

7. A process as claimed in any one of claims 1 to 6, wherein the product yield is about 64%.

## Claims (Claims for the following Contracting State(s): CH)

1. A process for the preparation of 2-chloro-5-methylpyridine-3-carbaldehyde which comprises reacting N-benzyl-N-(1-propenyl) acetamide with Vilsmeier reagent in a molar ratio of N-benzyl-N-(1-propenyl) acetamide to Vilsmeier reagent in the range of 1:17.5 to 1:5 at a first temperature in the range of 0-25°C for 1-4 hrs, and heating the resulting reaction mixture at a second temperature in the range of 70°C - 100°C for 4-6 hrs.

2. A process as claimed in claim 1 comprising extracting the reaction product of the heating stage with methylene chloride, separating the organic layer and removing the solvent to obtain the desired product.

3. A process as claimed in claim 1 or claim 2, wherein the mole ratio of N-benzyl - N-(1-propenyl) acetamide to Vilsmeier reagent is about 1:7.

4. A process as claimed in any one of claims 1 to 3, wherein N-benzyl -N-(1-propenyl) acetamide and Vilsmeier reagent are reacted at the first temperature for 2 hours.

5. A process as claimed in any one of claims 1 to 4, wherein the second temperature is in the range of 75-90 °C.

6. A process as claimed in any one of claims 1 to 5, wherein the first temperature is in the range of 0-10 °C.

7. A process as claimed in any one of claims 1 to 6, wherein the product yield is about 64%.

## Patentansprüche (Patentansprüche für folgende(n) Vertragsstaat(en): DE, FR, GB)

1. Verfahren zum Herstellen von 2-Chlor-5-Methylpyridin-3-Carbaldehyd, umfassend das Reagieren von N-Benzyl-N-(1-Propenyl)acetamid mit Vilsmeier-Reagenz in einem Molverhältnis von N-Benzyl-N-(1-Propenyl)acetamid zu Vilsmeier-Reagenz im Bereich von 1:17,5 bis 1:5 bei einer Temperatur im Bereich von 0-25°C für 1-4 Stunden, und Heizen der hervorgehenden Reaktionsmischung auf eine zweite Temperatur im Bereich von 70-100°C für 4-6 Stunden, wobei nicht eingeschlossen ein Verfahren ist umfassend Reagieren von N-Benzyl-N-(1-Propenyl)acetamid mit einem Reagenz ausgewählt aus Dimethylformamid gemischt mit Diphosgen oder Triphosgen unter trockenen Bedingungen bei einer Temperatur im Bereich von 75-100°C.

2. Verfahren nach Anspruch 1, umfassend Extrahieren des Reaktionsproduktes des Heizschrittes mit Methylenchlorid, Abtrennen der organischen Schicht und Entfernen des Lösungsmittels zum Erhalten des gewünschten Produkts.

3. Verfahren nach Anspruch 1 oder Anspruch 2, wobei das Molverhältnis von N-Benzyl-N-(1-Propenyl)acetamid zu Vilsmeier-Reagenz etwa 1:7 beträgt.

4. Verfahren nach einem der Ansprüche 1 bis 3, wobei N-Benzyl-N-(1-Propenyl)acetamid und Vilsmeier-Reagenz reagiert werden bei der ersten Temperatur für 2 Stunden.

5. Verfahren nach einem der Ansprüche 1 bis 4, wobei die zweite Temperatur im Bereich vom 75-90°C liegt.

6. Verfahren nach einem der Ansprüche 1 bis 5, wobei die erste Temperatur im Bereich von 0-10°C liegt.

7. Verfahren nach einem der Ansprüche 1 bis 6, wobei die Produktausbeute etwa 64% beträgt.

## Patentansprüche (Patentansprüche für folgende(n) Vertragsstaat(en): CH)

1. Verfahren zum Herstellen von 2-Chlor-5-Methylpyridin-3-Carbaldehyd, umfassend Reagieren von N-Benzyl-N-(1-Propenyl)acetamid mit Vilsmeier-Reagenz in einem Molverhältnis von N-Benzyl-N-(1-Propenyl)acetamid zu Vilsmeier-Reagenz im Bereich von 1:17,5 bis 1:5 bei einer ersten Temperatur im Bereich von 0-25°C für 1-4 Stunden, und Heizen der hervorgehenden Reaktionsmischung auf eine zweite Temperatur im Bereich von 70-100°C für 4-6 Stunden.

2. Verfahren nach Anspruch 1, umfassend Extrahieren des Reaktionsproduktes des Heizschrittes mit Methylenchlorid, Abtrennen der organischen Schicht und Entfernen des Lösungsmittels zum Erhalten des gewünschten Produkts.

3. Verfahren nach Anspruch 1 oder Anspruch 2, wobei das Molverhältnis von N-Benzyl-N-(1-Propenyl)acetamid zu Vilsmeier-Reagenz etwa 1:7 beträgt.

4. Verfahren nach einem der Ansprüche 1 bis 3, wobei N-Benzyl-N-(1-Propenyl)acetamid und Vilsmeier-Reagenz reagiert werden bei der ersten Temperatur für 2 Stunden.

5. Verfahren nach einem der Ansprüche 1 bis 4, wobei die zweite Temperatur im Bereich vom 75-90°C liegt.

6. Verfahren nach einem der Ansprüche 1 bis 5, wobei die erste Temperatur im Bereich von 0-10°C liegt.

7. Verfahren nach einem der Ansprüche 1 bis 6, wobei die Produktausbeute etwa 64% beträgt.

## Revendications

1. Procédé pour la préparation de 2-chloro-5-méthylpyridine-3-carbaldéhyde qui comprend la réaction de N-benzyl-N-(1-propényl)acétamide avec un réactif de Vilsmeier dans un rapport molaire du N-benzyl-N-(1-propényl)acétamide au réactif de Vilsmeier dans l'intervalle de 1:17,5 à 1:5 à une première température dans l'intervalle de 0-25°C pendant 1-4 h, et le chauffage du mélange réactionnel résultant à une seconde température dans l'intervalle de 70-100°C pendant 4-6 h, mais ne comprenant pas un procédé comprenant la réaction de N-benzyl-N-(1-propényl)acétamide avec un réactif choisi parmi du diméthyl-formamide mélangé avec du diphosgène ou du triphosgène dans des conditions sèches à une température dans l'intervalle de 75-100°C.

2. Procédé selon la revendication 1 comprenant l'extraction du produit de la réaction de l'étape de chauffage avec du chlorure de méthylène, la séparation de la couche organique et l'élimination du solvant pour obtenir le produit souhaité.

3. Procédé selon la revendication 1 ou la revendication 2, dans lequel le rapport molaire du N-benzyl-N-(1-propényl)acétamide au réactif de Vilsmeier est d'environ 1:7.

4. Procédé selon l'une quelconque des revendications 1 à 3, dans lequel le N-benzyl-N-(1-propényl)acétamide et le réactif de Vilsmeier réagissent à la première température pendant 2 heures.

5. Procédé selon l'une quelconque des revendications 1 à 4, dans lequel la seconde température se trouve dans l'intervalle de 75-90°C.

6. Procédé selon l'une quelconque des revendications 1 à 5, dans lequel la première température se trouve dans l'intervalle de 0-10°C.

7. Procédé selon l'une quelconque des revendications 1 à 6, dans lequel le rendement du produit est d'environ 64 %.

## Revendications (Revendications pour l'(les) Etat(s) contractant(s) suivant(s): CH)

1. Procédé pour la préparation de 2-chloro-5-méthylpyridine-3-carbaldéhyde qui comprend la réaction de N-benzyl-N-(1-propényl)acétamide avec un réactif de Vilsmeier dans un rapport molaire du N-benzyl-N-(1-propényl)acétamide au réactif de Vilsmeier dans l'intervalle de 1:17,5 à 1:5 à une première température dans l'intervalle de 0-25°C pendant 1-4 h, et le chauffage du mélange réactionnel résultant à une seconde température dans l'intervalle de 70-100°C pendant 4-6 h.

2. Procédé selon la revendication 1 comprenant l'extraction du produit de la réaction de l'étape de chauffage avec du chlorure de méthylène, la séparation de la couche organique et l'élimination du solvant pour obtenir le produit souhaité.

3. Procédé selon la revendication 1 ou la revendication 2, dans lequel le rapport molaire du N-benzyl-N-(1-propényl)acétamide au réactif de Vilsmeier est d'environ 1:7.

4. Procédé selon l'une quelconque des revendications 1 à 3, dans lequel le N-benzyl-N-(1-propényl)acétamide et le réactif de Vilsmeier réagissent à la première température pendant 2 heures.

5. Procédé selon l'une quelconque des revendications 1 à 4, dans lequel la seconde température se trouve dans l'intervalle de 75-90°C.

6. Procédé selon l'une quelconque des revendications 1 à 5, dans lequel la première température se trouve dans l'intervalle de 0-10°C.

7. Procédé selon l'une quelconque des revendications 1 à 6, dans lequel le rendement du produit est d'environ 64 %.
